# EUROPEAN PATENT APPLICATION

(11) **EP 1 481 689 A1**
(43) Date of publication of application: **01.12.2004**
(21) Application number: 03703269.5
(22) Date of filing: 07.02.2003
(51) Int. Cl.: A61K 45/00, A61K 31/445, A61K 7/06, A61K 9/06, A61K 9/08, A61K 9/70, A61P 17/14, A61P 43/00, C07D 211/32

(54) **HAIR GROWTH STIMULANTS, PERCUTANEOUS PREPARATIONS AND METHOD OF STIMULATING HAIR GROWTH**

(30) Priority: 07.02.2002 JP 2002031582
(71) Applicant: Eisai Co., Ltd., Tokyo 112-8088 (JP)
(72) Inventor: MIZUNO, Mitsuru, Ichinomiya-shi, Aichi 491-0053 (JP); KATO, Akira, kagamihara-shi, Gifu 509-0131 (JP); KATO, Takashi, Ichinomiya-shi, Aichi 491-0000 (JP)
(74) Representative: HOFFMANN EITLE
(86) International application number: PCT/JP2003/001332
(87) International publication number: WO 2003/066100

(57) **Abstract**

According to the present invention, a compound having a cholinesterase inhibiting action has a hair growth promoting action, and there is provided a hair growth promoting agent containing such a compound having a cholinesterase inhibiting action. Moreover, a compound having an acetylcholinesterase inhibiting action as the cholinesterase inhibiting action is preferable, and in particular donepezil hydrochloride has a good hair growth promoting effect. Furthermore, according to the present invention, there is provided a percutaneously applied preparation having a hair growth promoting action, containing a compound having a cholinesterase inhibiting action such as donepezil; the percutaneously applied preparation is preferably a liquid preparation, a cream, an ointment, a plaster or a tape preparation: Moreover, according to the present invention, there is also provided a scalp hair growth promoting method comprising the step of applying donepezil onto the scalp.

## Description

### Technical Field

The present invention relates to a hair growth promoting agent and a percutaneously applied preparation containing a compound having a cholinesterase inhibiting action, and a hair growth promoting method.

### Background Art

Cholinesterase inhibitors inhibit reduction in the amount of choline derivatives as a neurotransmitter in organisms. ln particular, an increase in the amount of acetylcholine, which is a neurotransmitter in the brain, is effective in improving the symptoms of senile dementia and dysmnesia, and out of cholinesterase inhibitors, donepezil hydrochloride is widely used as a therapeutic agent for Alzheimer's type senile dementia (see Patent Document 1).

On the other hand, as is well known, many cosmetics and medicinal agents that claim to conserve the head hair or have a hair growth promoting action are available on the market.

However, the effects of commercially available hair growth promoting agents are not sufficient, and a medicinal agent having excellent effects is eagerly awaited.

In view of the above state of art, it is thus a first object of the present invention to provide a useful hair growth promoting agent.

Moreover, it is a second object of the present invention to provide a useful percutaneously applied preparation having a hair growth promoting action.

Furthermore, it is a third object of the present invention to provide a useful hair growth promoting method.

### [Patent Document 1]

Japanese Patent Application Laid-open No. 1-79151

### Disclosure of Invention

The present inventors carried out assiduous studies with an aim of discovering a useful hair growth promoting agent, and as a result, completely unexpectedly, discovered that a compound having a cholinesterase inhibiting action has a hair growth promoting action, thus accomplishing the present invention.

That is, the above-mentioned first object is attained through a hair growth promoting agent, containing a compound having a cholinesterase inhibiting action or a pharmacologically acceptable sail thereof.

ln a preferable aspect of the hair growth promoting agent according to the present invention, the compound having a cholinesterase inhibiting action is a compound having an acetylcholinesterase inhibiting action.

In a preferable aspect of the hair growth promoting agent according to the present invention, the compound having an acetylcholinesterase inhibiting action is donepezil.

In a preferable aspect of the hair growth promoting agent according to the present invention, the pharmacologically acceptable salt of the compound having a cholinesterase inhibiting action is a hydrochloride.

Moreover, the above-mentioned second object is attained through a percutaneously applied preparation having a hair growth promoting action, containing a compound having a cholinesterase inhibiting action or a pharmacologically acceptable salt thereof.

In a preferable aspect of the percutaneously applied preparation according to the present invention, the compound having a cholinesterase inhibiting action is a compound having an acetylcholinesterase inhibiting action.

In a preferable aspect of the percutaneously applied preparation according to the present invention, the compound having an acetylcholinesterase inhibiting action is donepezil.

In a preferable aspect of the percutaneously applied preparation according to the present invention, the pharmacologically acceptable salt of the compound having a cholinesterase inhibiting action is a hydrochloride.

In a preferable.aspect of the percutaneously applied preparation according to the present invention, the percutaneously applied preparation is a liquid preparation, a cream, an ointment, a plaster or a tape preparation.

Furthermore, the above-mentioned third object is attained through a scalp hair growth promoting method, comprising the step of applying a compound having a cholinesterase inhibiting action or a pharmacologically acceptable salt thereof onto the scalp.

In a preferable aspect of the hair growth promoting method according to the present invention, the compound having a cholinesterase inhibiting action is a compound having an acetylcholinesterase inhibiting action.

In a preferable aspect of the hair growth promoting method according to the present invention, the compound having an acetylcholinesterase inhibiting action is donepezil.

In a preferable aspect of the hair growth promoting method according to the present invention, the pharmacologically acceptable salt of the compound having a cholinesterase inhibiting action is a hydrochloride.

Note that the term "hair growth promotion" used in the present invention means promotion of the elongation of hair on the body, in particular head hair. Moreover, this term "hair growth promotion" used herein is also referred to as "hair fostering promotion", and an effect of preventing hair from falling out is included in the meaning thereof.

### Brief Description of Drawings

FIG. 1 shows a formulation for manufacturing a tape preparation containing donepezil according to the present invention.
FIG. 2 shows results of an experiment regarding hair growth promoting action using rats according to the present invention.

### Best Mode for Carrying Out the Invention

Following is a detailed description of the hair growth promoting agent, the percutaneously applied preparation, and the hair growth promoting method according to the present invention.

In the present invention, "compound having a cholinesterase inhibiting action" means a substance that inhibits the activity of a cholinesterase; cholinesterases include butylcholinesterase, acetylcholinesterase and so on. As compounds that inhibit acetylcholinesterase, donepezil, physostigmine, galantamine, rivastigmine and so on are known, but donepezil is particularly preferable. Donepezil is 1-benzyl-4-[(5,6-dimethoxy-1-indanon)-2-yl]methylpiperidine, represented by undermentioned formula (I), and may be in the form of a pharmacologically acceptable salt.

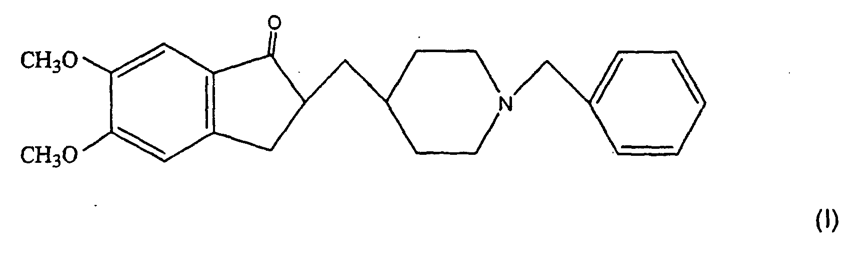

Regarding the method of manufacturing the donepezil used in the present invention, the donepezil can easily be manufactured following a publicly known method, selecting the reaction raw materials, reaction reagents, reaction conditions and so on as appropriate. For example, the donepezil can be manufactured through the method described in Japanese Patent Application Laid-open No. 1-79151.

Examples of pharmacologically acceptable salts in the present invention include salts with inorganic acids and salts with organic acids. A salt is formed with the acid in a suitable proportion of 0.1 to 5 molecules per molecule of the compound in question.

Preferable examples of salts with inorganic acids include salts with hydrochloric acid, hydrobromic acid, sulfuric acid, nitric acid, and phosphoric acid. Preferable examples of salts with organic acids include salts with acetic acid, succinic acid, fumaric acid, maleic acid, tartaric acid, citric acid, lactic acid, stearic acid, benzoic acid, methanesulfonic acid, and p-toluenesulfonic acid.

Moreover, preferable examples of salts with organic bases include salts with diethylamine, diethanolamine, meglumine, and N,N'-dibenzylethylenediamine.

In the present invention, an active ingredient that is particularly preferable from the viewpoint of hair growth promotion is donepezil hydrochloride, represented by undermentioned formula (II).

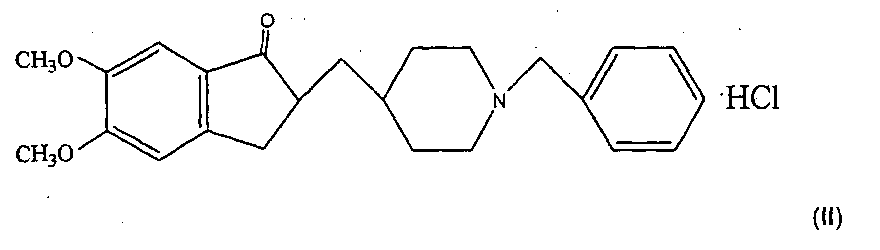

Note that the donepezil used in the present invention has an asymmetric carbon, and hence may exist as an optically active substance, but in the present invention any of a freely chosen mixture of optically active substances or stereoisomers such as diastereoisomers, or a racemate, or the like may be used.

In the present invention, the dose of the compound having a cholinesterase inhibiting action or the like will vary according to the type of the compound and the form of the preparation and hence it is not possible to make a sweeping generalization, but the dose will generally be 0.1 to 100 mg per time, and in the case of applying donepezil or the like onto the scalp, the dose will generally be 0.1 to 100 mg, preferably 1 to 50 mg, more preferably 5 to 50 mg, per time.

The hair growth promotion targeted by the present invention is the promotion of the elongation of hair on the body, in particular head hair, and here it is preferable for the compound having a cholinesterase inhibiting action such as donepezil to be applied to the head hair percutaneously, although other methods such as taking as an internal medicine may also be used. There are no particular limitations on the form of the preparation in the case of percutaneous application, but a liquid preparation, a cream, an ointment, a plaster or a tape preparation is preferable.

The liquid preparation, cream, ointment, plaster, tape preparation or the like containing the compound such as donepezil can be manufactured using an ordinary method; for example, in the case of a liquid preparation, the liquid preparation can be manufactured by dissolving the donepezil or the like in water or in water containing an organic solvent such as ethanol, and as necessary adding absorption promoters, stabilizers, pH regulators, preservatives, perfumes and so on using ordinary methods.

Note that tests of toxicity in rats were carried out on donepezil which is a compound used in the present invention, whereupon serious toxicity was not exhibited at a dose of approximately 100 mglkg or more.

As shown in the following examples, hair growth is markedly promoted by donepezil, and hence the present invention also provides a scalp hair growth promoting method comprising applying donepezil onto the scalp.

### Examples

Following is a detailed description of the hair growth promoting action according to the present invention, with reference to preferable examples of the present invention; however, the present invention is not limited to these examples whatsoever.

### Example of manufacture of tape preparation

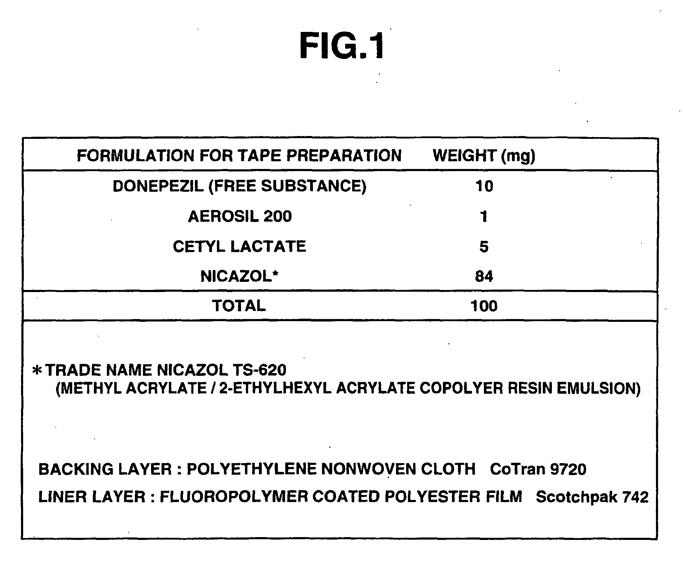

First, a description of a tape preparation used in the present invention will be given. FIG. 1 shows a formulation for manufacturing the tape preparation according to the present invention. The formulation shown in FIG. 1 was weighed out precisely into a glass vial, an appropriate amount of water was added and dilution and stirring were carried out, and then ultrasound treatment was carried out to obtain a homogeneous solution. The homogeneous solution was applied uniformly onto a 20 cm × 15 cm polyethylene nonwoven cloth.

Afterthere, drying was carried out overnight at 40°C, and then a fluoropolymer coated polyester film was put on, thus obtaining the tape preparation used in the present invention.

The tape preparation obtained as described above was cut into 4 cm × 3 cm pieces, thus preparing an active tape preparation. SD-type SPF rats were used as test animals. Note that the rats used in the experiment were 10 weeks or 8 weeks old.

On the day before the experiment, both the 10-week-old rats and the 8-week-old rats were anesthetized with ether, and then the hair on the back was shaved off using electric clippers and an electric shaver. Furthermore, on the day of the experiment, hair was again removed from the hair-removed sites using an electric shaver.

After checking that there were no abnormalities on the skin where the tape preparation was to be stuck on, the skin was wiped clean with cotton wool containing 70% ethanol to remove sebum from the skin, and then two pieces of the active tape preparation were stuck onto the skin on the left and right of the back of each of the ether-anesthetized rats.

Moreover, for a lotion as a liquid preparation, the ingredients of the above-mentioned active tape prepration were stuck/applied onto gauze, covering with Parafilm was carried out, and then fixing with an adhesive elastic bandage was carried out.

After the tape had been stuck on, the tape was fixed for 72 hours, and then the tape was peeled off, and the base material was wiped up. Note that for a placebo tape, the base material only of the above-mentioned tape preparation was used.

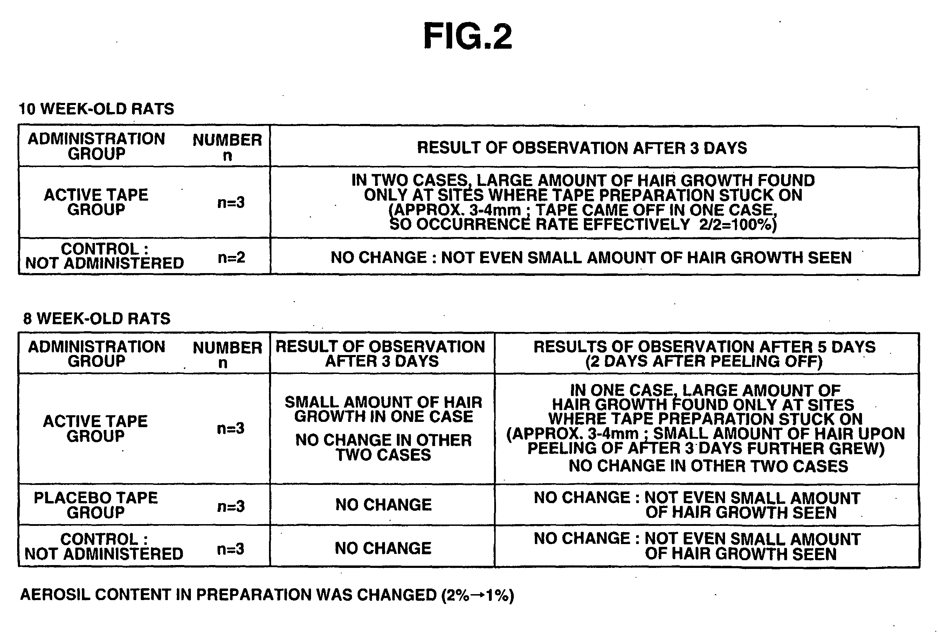

FIG. 2 shows the results of the experiment into hair growth promoting action using the 10-week-old and 8-week-old rats. With the experimental group of 10-week-old rats, in one case while the tape preparation was stuck on, the tape preparation came off, and hence the hair growth effect could not be verified, but for the other two cases, a large amount of hair growth was found from the results of visual observation at the parts where the tape preparation had been stuck on. In contrast with this, with a 10-week-old control group for which donepezil was not administered, no hair growth was observed at all at the hair-removed sites on the rats. The experimental results for the 10-week-old rats thus show that the hair growth at the parts where the tape preparation was stuck on was not natural hair growth, but rather was due to the action of the donepezil contained in the tape preparation.

On the other hand, with the experimental group of 8-week-old rats, after the tape preparation had been stuck on for 72 hours, the tape preparation was peeled off, and then two days after that the hair-removed sites were observed. The result was that for the active tape (donepezil-containing tape) group, in one case a small amount of hair growth was observed after peeling off the tape preparation, and two days after that much hair growth was observed. In contrast with this, with the placebo tape group and a group for which donepezil was not administered as a control, hair growth promotion at the hair-removed sites was not observed whatsoever.

Moreover, similar results were also obtained for the lotion. ln Kiso To Rinsho §1 (11), 3217-3220, 1997, it is disclosed that an increase in blood flow contributes to hair growth promotion, and it is conjectured that promotion of blood flow is also contributing in the case of the effects of the present invention.

From the above, it was ascertained that donepezil, which is a compound having a cholinesterase inhibiting action, has an excellent hair growth action.

Moreover, a person skilled in the art in the technical field in question could readily appreciate that there would also be a hair growth promoting action in the case of sticking a tape preparation containing donepezil onto the scalp.

### Industrial Applicability

According to the present invention, there are provided a hair growth promoting agent and a percutaneously applied preparation having as an active ingredient thereof a compound having a cholinesterase inhibiting action or a pharmacologically acceptable salt thereof, and a hair growth promoting method; the present invention can be used in the protection of head hair and so on.

## Claims

1. A hair growth promoting agent, comprising a compound having a cholinesterase inhibiting action or a pharmacologically acceptable salt thereof.

2. The hair growth promoting agent according to claim 1, wherein said compound having a cholinesterase inhibiting action is a compound having an acetylcholinesterase inhibiting action.

3. The hair growth promoting agent according to claim 2, wherein said compound having an acetylcholinesterase inhibiting action is donepezil.

4. The hair growth promoting agent according to claim 1, wherein said pharmacologically acceptable salt of said compound having a cholinesterase inhibiting action is a hydrochloride.

5. A percutaneously applied preparation having a hair growth promoting action, comprising a compound having a cholinesterase inhibiting action or a pharmacologically acceptable salt thereof.

6. The percutaneously applied preparation according to claim 5, wherein said compound having a cholinesterase inhibiting action is a compound having an acetylcholinesterase inhibiting action.

7. The percutaneously applied preparation according to claim 6, wherein said compound having an acetylcholinesterase inhibiting action is donepezil.

8. The percutaneously applied preparation according to claim 5, wherein said pharmacologically acceptable salt of said compound having a cholinesterase inhibiting action is a hydrochloride.

9. The percutaneously applied preparation according to any one of claims 5 through 8, wherein said percutaneously applied preparation is a liquid preparation, a cream, an ointment, a plaster or a tape preparation.

10. A scalp hair growth promoting method, comprising the step of applying a compound having a cholinesterase inhibiting action or a pharmacologically acceptable salt thereof onto the scalp.

11. The hair growth promoting method according to claim 10, wherein said compound having a cholinesterase inhibiting action is a compound having an acetylcholinesterase inhibiting action.

12. The hair growth promoting method according to claim 11, wherein said compound having an acetylcholinesterase inhibiting action is donepezil.

13. The hair growth promoting method according to claim 10, wherein said pharmacologically acceptable salt of said compound having a cholinesterase inhibiting action is a hydrochloride.
